(19) Europäisches Patentamt
European Patent Office
Office européen des brevets

(11) **EP 3 032 226 B1**

(12) **EUROPÄISCHE PATENTSCHRIFT**

(45) Veröffentlichungstag und Bekanntmachung des Hinweises auf die Patenterteilung:
**05.07.2017 Patentblatt 2017/27**

(51) Int Cl.:
**G01F 1/66** *(2006.01)*      **G01N 29/024** *(2006.01)*
**G01N 33/00** *(2006.01)*     **G01F 15/04** *(2006.01)*

(21) Anmeldenummer: **14197704.1**

(22) Anmeldetag: **12.12.2014**

(54) **Verfahren und Ultraschalldurchflussmessgerät zur Bestimmung des CO2-Emmissionsfaktors bei Fackelgasanlagen**

Method and ultrasound flow measuring device for determining the CO2 emission factor in flare gas systems

Procédé et débitmètre à ultrasons destinés à la détermination du facteur d'émission de CO2 pour des installations à gaz brûlé à la torche

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.06.2016 Patentblatt 2016/24**

(73) Patentinhaber: **SICK Engineering GmbH 01458 Ottendorf-Okrilla (DE)**

(72) Erfinder:
• **Riedel, Ekkehard 01109 Dresden (DE)**
• **Nerowski, Alexander 01277 Dresden (DE)**

(74) Vertreter: **Ludewigt, Christoph Sick AG Intellectual Property Erwin-Sick-Strasse 1 79183 Waldkirch (DE)**

(56) Entgegenhaltungen:
EP-A1- 2 330 347          EP-A2- 1 063 525
DE-U1-202006 021 163     US-A1- 2003 082 821
US-B1- 6 216 091          US-B2- 7 752 884

**Beschreibung**

Die Erfindung betrifft ein Verfahren zur Bestimmung des CO2-Emissionsfaktors bei Fackelgasanlagen sowie ein Ultraschalldurchflussmessgerät, mit dem dieses Verfahren durchführbar ist.

Aufgrund neuer EU-Richtlinien (z. B. Richtlinie 2003/87/EG des Europäischen Parlaments und des Rates vom 26.02.2004, bekanntgegeben unter Aktenzeichen K(2004) 130 und veröffentlicht im Amtsblatt der Europäischen Union L 59/1 bis L59/74) und teilweise auch nationaler Richtlinien (z. B. in Norwegen) müssen auf CO2-Emissionen Steuern gezahlt werden. Da sich die Steuerlast erhöht je ungenauer die CO2-Emissionen bekannt sind, ist es nötig, die zeitlich akkumulierten CO2-Emissionen möglichst genau kontinuierlich zu überwachen. Insbesondere bei Fackelgasanlagen werden Lösungen gesucht, da eine Messung des bei der **Verbrennung des Gases (auch "Fackelgas" oder "Flaregas" genannt) entstehende** CO2 sehr schwer ist, da die Verbrennung an offener Flamme erfolgt.

Der CO2-Gehalt eines Gases kann mit verschiedenen Methoden gemessen werden. Dazu zählen u. a. die Fourier-Transform-Infrared (FTIR)-Spektrometrie, die Gaschromatographie und die Flammenionisationsdetektion (FID).

Die FTIR-Spektrometrie ist ein optisches Verfahren, bei dem mit Infrarotlicht (Wellenlängen von 800 nm bis 1 mm) das Gas bestrahlt wird und die Absorption gemessen wird. Die Wellenlänge(n), bei der/denen absorbiert wird, ist/sind molekülspezifisch und die Intensität der Absorption korreliert mit dem molaren Anteil des Moleküls am Gesamtgas. Das Verfahren funktioniert nur bei Molekülen, die eine IR-aktive Bindung haben, was aber auf CO2 zutrifft.

In Flaregasanwendungen sind mit einem FTIR-Gerät zwei Applikationsorte denkbar, nämlich vor der Flamme und nach der Flamme. Da man aber in Fackelgaspipelines mit starker Verschmutzung zu kämpfen hat, sind optische Lösungen vor der Flamme nicht praktikabel, weil die direkt mit dem Gas in Kontakt stehenden transparenten Teile nach einiger Operationszeit nicht mehr lichtdurchlässig sind. Den CO2-Ausstoß nach der Flamme zu messen, ist mit erheblichen Schwierigkeiten verbunden, da das verbrannte Gas völlig frei in die Atmosphäre diffundiert.

Bei der Gaschromatographie werden die Einzelbestandteile einer Gasmischung nach ihrem Siedepunkt und ihrer Polarität aufgetrennt und einzeln gemessen. Nachteilig daran ist, dass die Gaschromatographie ein sehr träges Verfahren ist, weswegen eine kontinuierliche Überwachung nicht möglich ist. Zudem sind Gaschromatographen sehr kostenaufwändig in der Anschaffung.

Bei der Flammionisationsdetektion (FID) wird das zu analysierende Gas an einer Wasserstoffflamme verbrannt. Die entstehenden Ionen werden mit Hilfe einer Beschleunigungsspannung von einer Elektrode abgesaugt, so dass ein Strom messbar ist, der proportional zum Gesamtgehalt an Kohlenwasserstoffen des Gases ist.

Ein FID kann nur Kohlenwasserstoffe detektieren, weswegen das Gerät vor der Flamme platziert werden müsste, da nach der Flamme die Kohlenwasserstoffe zersetzt sind. Ein FID-Gerät ist aber aufgrund seiner Wasserstoffflamme nicht geeignet für explosionsgefährdete Bereiche und kommt daher nicht für Messungen vor der Fackelflamme in Frage, wo der Methananteil des Gases oft um die 90 % liegt. Eine Messung nach der Flamme kommt nicht in Frage, da nach der Verbrennung keine Kohlenwasserstoffe mehr im Gas enthalten sind und das Gerät auf andere Gase nicht sensitiv ist.

All diesen drei Lösungen ist gemein, dass man ein zusätzliches Gerät an der Gasleitung installieren muss, das den Volumenstrom misst, um die zeitlich akkumulierten CO2-Emissionen zu erhalten.

Aus der US 7,752,884 ist ein Verfahren bekannt, um die Zusammensetzung eines Gases, insbesondere den Energieinhalt, zu analysieren. Es wird eine hypothetische prozentuale Zusammensetzung von Kohlenwasserstoffen genommen und davon die Schallgeschwindigkeit errechnet. Die hypothetischen Zusammensetzungen sind sehr vielfältig und enthalten stets eine Vielzahl Alkane. Für diese künstlichen Zusammensetzungen werden jeweils die Schallgeschwindigkeiten berechnet und mit einer gemessenen Schallgeschwindigkeit verglichen. Bei Übereinstimmung der Schallgeschwindigkeiten innerhalb einer gewissen Toleranz wird angenommen, dass die hypothetische Zusammensetzung der wirklichen entspricht, so dass der Energieinhalt errechnet werden kann.

Weiterer Stand der Technik ist von Froysa et. al. bekannt (Konferenzband "28th International North Sea Flow Measurement Workshop 2010", ISBN: 978-1-61782-294-0, Publ: Energy Institute, POD Publ: Curran Associates, Inc. (Apr. 2011), Seite 145, "A Cost-Effective Approach on CO2 Emission Factor Estimation for Flare Ultrasonic Metering Systems" von Jeff Gibson, Richard Paton, Pal Jagho, Kjell-Eivind Froysa, Anders Hallanger, Endre Jacobsen und Anders Lovoll Johansen). Hier wird beschrieben, wie aus bestimmten Eingangsparametern und einem mit einem Durchflussmessgerät bestimmten Durchfluss, die bei einer Verbrennung entstehenden CO2-Emissionen bestimmt werden können, wie nachfolgend kurz erläutert.

Die Gesamtmasse $m_{CO2}$ des emittierten CO2 setzt sich aus dem bei der Verbrennung der im Gas enthaltenen Kohlenwasserstoffe entstehenden CO2 und dem bereits im Gas vorhandenen CO2 zusammen.

$$m_{CO2}(gesamt) = m_{CO2}(aus\ Verbrennung) + m0_{CO2}(bereits\ im\ Gas\ vorhanden) \quad (1)$$

Nach den Leitlinien für Überwachung und Berichterstattung betreffend Treibhausgasemissionen gemäß der vorgenannten Richtlinie 2003/87/EG wird ein Emissionsfaktor e und ein Oxydationsfaktor $\Omega$ definiert. Diese Faktoren stellen ein Maß dafür dar, wieviel CO2 ($m_{CO2}$) bei der Verbrennung einer Menge $m_{Flare}$ des Gases entsteht, wobei der dimensionslose Oxydationsfaktor $\Omega$ angibt, welcher Anteil der im Flaregas enthaltenen Kohlenwasserstoffe verbrannt wird. Der Wert 1 entspricht einer vollen Verbrennung der Kohlenwasserstoffe zu CO2. Von den Anlagenbetreibern wird $\Omega$ mit hinreichender Genauigkeit zu 0,98 angegeben.

$$m_{CO2}(Verbrennung) = Emissionsfaktor\ e * m_{Flare} * \Omega \qquad (2)$$

$$=> e * \Omega = \frac{m_{CO2}}{m_{Flare}} \qquad (3)$$

Dieses Massenverhältnis kann auch molar ausgedrückt werden

$$=> e * \Omega = \frac{m_{CO2}}{m_{Flare}} = \frac{M_{CO2}}{M_{Flare}} * \sum_{i=1}^{N} f_i * n_i \qquad (4)$$

wobei $f_i$ der Anteil der i-ten Kohlenstoffverbindung ist und $n_i$ die Anzahl Kohlenstoffe in der i-ten Kohlenstoffverbindung und über alle im Flaregas vorkommenden Kohlenstoffverbindungen summiert wird.

Die im Flaregas auftretenden Kohlenstoffverbindungen sind im Prinzip unbekannt. Es wurde aber festgestellt, dass der Fehler akzeptabel bleibt, wenn man annimmt, dass diese nur als Alkane verschiedener Kettenlänge vorkommen, wobei die Kettenlänge normalerweise nicht größer 6 ist. Dann lässt sich mit einer sogenannten äquivalenten Kettenlänge n und dem Anteil der Kohlenwasserstoffe $f_{CH}$ die molare Masse $M_{Flare}$ und damit der Emissionsfaktor e berechnen. Es gelten für die äquivalente Kettenlänge:

$$n = \frac{\sum_{i=1}^{N} i f_{C_i H_{2i+2}}}{f_{CH}} \qquad (5)$$

und mit dieser äquivalenten Kettenlänge für die molare Masse der Kohlenwasserstoffe (Alkane):

$$M_{CH} = n M_C + (2n + 2) M_H \qquad (6)$$

Damit kann die molare Masse des Flaregases berechnet werden, wobei das Flaregas neben den Kohlenwasserstoffen noch Anteile an Stickstoff, Kohlendioxid und Wasser aufweist:

$$M_{Flare} = f_{CH} M_{CH} + f_{N2} M_{N2} + f_{H2O} M_{H2O} + f_{CO2} M_{CO2} \qquad (7)$$

Die verschiedenen Anteile $f_{N2}$, $f_{H2O}$, $f_{CO2}$ und $f_{CH}$ = 1 - ($f_{N2} + f_{H2O} + f_{CO2}$) werden aus Erfahrungswerten beim Betrieb einer Anlage gewonnen und als Schätzgrößen durch den Nutzer als Parameter vor der Berechnung eingegeben. Damit ergibt sich für die durch die Verbrennung entstehende Emission e* $\Omega$:

$$e * \Omega = \frac{M_{CO2}}{M_{Flare}} * (n f_{CH}) \qquad (8)$$

Insgesamt ergibt sich damit für den gesamten Massenstrom $\dot{m}$, also für den gesamten CO2 Ausstoß (Menge CO2) pro Zeit:

$$\dot{m}_{CO2}(gesamt) = \dot{m}_{CO2}(Verbrennung) + \dot{m0}_{CO2}(bereits\ im\ Gas)$$

$$= m_{Flare} * e * \Omega + m_{Flare} * f_{CO2}$$

$$= \varrho * \dot{Q} * e * \Omega + \varrho * \dot{Q} * f_{CO2}$$

$$= \varrho * \dot{Q} * (e * \Omega + f_{CO2}) \qquad\qquad (10)$$

wobei $\dot{Q}$ der Volumenstrom ist und e die Dichte des Gases. Die Dichte wird mit bekannten Algorithmen abgeschätzt durch eine gemessene Schallgeschwindigkeit. Der Volumenstrom wird mit einem Ultraschallmessgerät gemessen. Der Anteil $f_{CO2}$ am bereits vor der Verbrennung im Gas vorhandenen CO2 ist bekannt.

Damit lässt sich dann der CO2-Ausstoß bestimmen, wobei die Schallgeschwindigkeit und der Volumenstrom die einzigen Messgrößen bilden.

Die Erfindung greift dieses Prinzip, also Bestimmung des CO2-Ausstoßes über einen gemessenen Volumenstrom in Zusammenhang mit dem Gedanken der äquivalenten Kettenlänge zur Berechnung des Emissionsfaktors, auf. Dabei wird aber eine neue Lösung aufgezeigt, wie die Gaszusammensetzung besser ermittelt werden kann, um daraus ein zuverlässigeres Maß für die kontinuierliche Überwachung des CO2-Ausstoßes an einer Fackelgasleitung zu ermöglichen, ohne dass ein zusätzliches Gerät installiert werden muss.

Diese Aufgabe wird gelöst durch ein Verfahren mit den Merkmalen des Anspruchs 1 und einer Vorrichtung nach Anspruch 8.

Das erfindungsgemäße Verfahren zur Bestimmung des CO2-Emissionsfaktors bei Fackelgasanlagen umfasst die Schritte gemäß Anspruch 1. Dieses neue Verfahren enthält die wesentliche Kernidee, dass die Kohlenwasserstoffe des Fackelgases für die Berechnung des Emissionsfaktors **quasi ersetzt werden durch lediglich ein "passendes" Paar** Alkane mit benachbarten Kettenlängen, also mit Kettenlänge i und i+1.

Das **"passendste" Paar Alkane** wird durch eine erste Variation, vorzugsweise iterativ, gefunden, indem jeweils die Schallgeschwindigkeit des theoretischen Fackelgases berechnet wird, dessen Kohlenwasserstoffe jeweils nur das eine bzw. nur das andere Alkan aufweisen und die berechnete Schallgeschwindigkeit mit der real gemessenen verglichen wird. Liegt die gemessene Schallgeschwindigkeit zwischen den beiden berechneten, **ist das "passende Paar"** Alkane gefunden. Damit erhält man eine erste grobe Annäherung an die Zusammensetzung des Fackelgases.

Diese erfindungsgemäße Vorgehensweise vereinfacht die weitere numerische Bestimmung der Gaszusammensetzung erheblich, da die Anzahl von Variationsgraden für die Anteile der Kohlenwasserstoffe von sechs, wie im Stand der Technik nach Fröysa et al, auf zwei verringert wird. Diese Vereinfachung zur Überführung von Kohlenwasserstoffgemischen auf ein Gemisch aus nur zwei verschiedenen Alkanen, besitzt allerdings eine Unsicherheit hinsichtlich der Berücksichtigung der zwei Wasserstoffatome am Anfang und Ende der Molekülketten. Diese Unsicherheit hat sich aber in Bezug auf die Gesamtunsicherheit des Verfahrens als akzeptabel herausgestellt.

In einer zweiten Variation der Anteile der beiden so gefundenen Alkane werden die Anteile, vorzugsweise ausgehend von einem Anfangszustand, in dem der eine Anteil Null ist und der andere Anteil maximal, schrittweise variiert, bis die mit den jeweiligen Anteilen berechnete Schallgeschwindigkeit nahe genug (innerhalb eines Abbruchkriteriums) an der gemessenen Schallgeschwindigkeit liegt.

Hat man auf diese Art die Anteile gefunden, kann die äquivalente Kettenlänge und daraus der Emissionsfaktor berechnet werden. Über den mit einem Durchflussmessgerät gemessenen Gasdurchfluss und dem Oxidationsfaktor kann dann der CO2-Ausstoß berechnet werden.

Das erfindungsgemäße Verfahren liefert erstens genauere Ergebnisse, als die Verfahren nach dem Stand der Technik und zweitens ist es einfacher auszuführen. Im Prinzip besteht es nur aus zwei Phasen.

In der ersten Phase wird in einem vorzugsweise iterativen Algorithmus der Index i variiert und die Schallgeschwindigkeit für das nur noch Kohlenwasserstoffemoleküle zweier Kettenlängen aufweisende Gemisch in jedem Iterationsschritt berechnet. Für die Berechnung wird vorzugsweise der Algorithmus nach GERG-2004 XT08 verwendet **(siehe auch "**The GERG-2004 Wide-Range Equation of State for Natural Gases and Other Mixtures" von O. Kunz, R. Klimeck, W. Wagner, M. Jaeschke in GERG TECHNICAL MONOGRAPH 15 (2007), VDI Verlag GmbH, Düsseldorf 2007, Reprinted from Fortschritt-Berichte VDI, Reihe 6 Nr. 557 (2007), ISBN 978-3-18-355706-6). Alternativ können auch andere Verfahren, z. B. AGA report 10 (AGA Report No. 10, Speed of Sound in Natural Gas and Other Related Hydrocarbon Gases, January, 2003) oder GSSSD MR 113-03 (Hydrocarbon Gas), verwendet werden. Die Iterationsschleife wird so lange durchlaufen, bis die gemessene Schallgeschwindigkeit des Ultraschallgaszählers innerhalb der theoretischen Schallgeschwindigkeiten der Fackelgase liegt, die **nur "reine" Alkane mit einer einzigen Ket**tenlänge i bzw. i+1 aufweisen.

In der zweiten Phase wird das Mischungsverhältnis der beiden Alkane mit Kettenlänge i und i+1 bestimmt.

Eine geeignete Vorrichtung zur Implementierung des Verfahrens bildet erfindungsgemäß ein Ultraschalldurchflussmessgerät, mit einer Eingabeeinheit zum Eingeben der bekannten N2-, CO2- und H2O-Anteile des Fackelgases, mit Ultraschallwandlern zum Aussenden und Empfangen von Ultraschallsignalen und mit einer Auswerteeinheit, die ausgebildet ist, um eine Schallgeschwindigkeit aus den Ultraschallsignalen zu bestimmen und um die Schritte a) bis h) des Verfahrens auszuführen und um den Gasdurchfluss über die gemessene Strömungsgeschwindigkeit zu bestimmen, wobei die Strömungsgeschwindigkeit aus der Laufzeitdifferenz zweier Ultraschallsignale ermittelbar ist, die einmal eine Komponente mit und einmal eine Komponente gegen die Strömung aufweisen.

Eine solche Vorrichtung kann also nicht nur das erfindungsgemäße Verfahren ausführen, sondern auch die Messung der Schallgeschwindigkeit des Fackelgases vornehmen.

Mit dem erfindungsgemäßen Verfahren und Vorrichtung lässt sich letztendlich mit Gasdurchfluss und Emissionsfaktor der CO2-Ausstoß genauer berechnen und kontinuierlich ausgeben.

Im Folgenden wird die Erfindung anhand eines Ausführungsbeispiels unter Bezugnahme auf die Zeichnung im Einzelnen erläutert. In der Zeichnung zeigen:

Fig. 1 ein beispielhaftes Ablaufdiagramm des erfindungsgemäßen Verfahrens;

Fig. 2 eine schematische Darstellung eines erfindungsgemäßen Ultraschalldurchflussmessgeräts.

Zunächst soll das erfindungsgemäße Verfahren beispielhaft beschrieben werden.

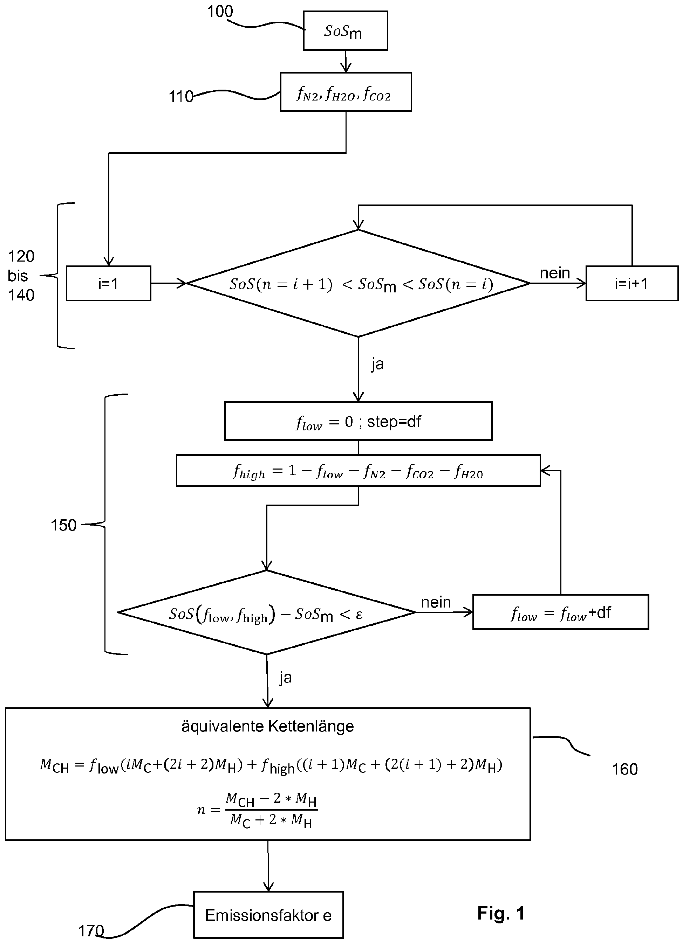

Fig. 1 zeigt ein Ablaufdiagramm. In einem ersten Schritt 100 (auch mit a) bezeichnet) wird die Schallgeschwindigkeit $SoS_m$ (Speed of Sound) im Fackelgas gemessen.

Ein Fackelgas setzt sich typischerweise aus verschiedenen Gasen zusammen, von denen die Kohlenwasserstoffe (CH) bei der Verbrennung in CO2 umgewandelt werden. Die anderen Bestandteile, die bei einer Verbrennung nicht zu CO2 umgewandelt werden, sind Stickstoff (N2), Wasser (H2O) und Kohlendioxid (CO2).

Die Anteile der inerten Bestandteile sind in der Regel bekannt und werden in einem zweiten Schritt 110 (auch mit b) bezeichnet) erfasst, z. B. indem sie manuell eingegeben werden.

Unbekannt sind hingegen die Anteile der verschiedenen Kohlenwasserstoffe, die aber durch ihre Verbrennung zu CO2 den CO2-Ausstoß bei der Verbrennung des Fackelgases entscheidend beeinflussen.

Um hier eine bessere Abschätzung zu erhalten, wird das eingangs beschriebene und von Fröysa et al beschriebene Prinzip angewendet, wonach für die Kohlenwasserstoffe angenommen wird, dass diese nur als Alkane vorkommen. Die Erfindung geht nun noch weiter und nimmt an, dass nur zwei Typen Alkane vorkommen und zwar solche, die benachbarte Kettenlänge haben, also der eine Kohlenwasserstoffanteil ist ein Alkan mit der Kettenlänge i mit einem Anteilsfaktor $f_{low}$ und das andere mit der Kettenlänge i+1 und einem Anteilsfaktor $f_{high}$. Die molare Masse des Kohlenwasserstoffs in dem Fackelgas ist dann:

$$M_{CH} = f_{low}(i M_C + (2i + 2)M_H) + f_{high}((i + 1)M_C + (2(i + 1) + 2)M_H) \qquad (11)$$

Dann wird in den nächsten drei Schritten 120, 130 und 140 (auch mit c1), c2) undc3) bezeichnet) die Kettenlänge i bestimmt. Dies geschieht in diesem Ausführungsbeispiel durch Iteration, beginnend mit i=1.

Im ersten Teilschritt 120 wird eine maximale Schallgeschwindigkeit $SoS(f_{high} = 0)$ des Fackelgases berechnet. Dabei wird angenommen, dass nur Alkane mit Kettenlänge i vorkommen, also $f_{high} = 0$ und $f_{low} = 1 - (f_{CO2} + f_{H2O} + f_{N2})$. Die Berechnung der Schallgeschwindigkeit $SoS(f_{high} = 0)$ erfolgt nach dem Algorithmus GERG-2004 XT08.

Im zweiten Teilschritt 130 wird eine minimale Schallgeschwindigkeit $SoS(f_{low} = 0)$ des Fackelgases berechnet. Dabei wird angenommen, dass nur Alkane mit Kettenlänge i+1 vorkommen, also $f_{low} = 0$ und $f_{high} = 1 - (f_{CO2} + f_{H2O} + f_{N2})$.

Im dritten Teilschritt 140 schließlich wird geprüft, ob die gemessene Schallgeschwindigkeit zwischen den beiden Berechneten liegt.

$$SoS(f_{low} = 0;\ n = i + 1)\ < SoS_m < SoS(f_{high} = 0;\ n = i) \qquad (12)$$

Die Teilschritte 120, 130 und140 werden iterativ jeweils mit Erhöhung der Kettenlänge i um 1 so lange durchlaufen, bis die gemessene Schallgeschwindigkeit zwischen den beiden Berechneten liegt. Dann ist das "passende Paar" Alkane gefunden.

Im nächsten Schritt 150 (auch mit d) bezeichnet) werden die Anteile, also $f_{low}$ und $f_{high}$, der beiden Alkane bestimmt. Dies geschieht, indem wieder in einer Iteration ausgehend von $f_{low} = 0$ (oder alternativ von $f_{high} = 0$) in festgelegten Schritten der Größe df $f_{low}$ und $f_{high}$ geändert werden (wobei wie oben gilt 1 = $f_{low}$ + $f_{high}$ + $f_{CO2}$ + $f_{H2O}$ + $f_{N2}$) und in jedem Iterationsschritt die Schallgeschwindigkeit SoS berechnet wird und mit der gemessenen $SoS_m$ verglichen wird. Liegen berechnete und gemessene Schallgeschwindigkeiten weniger als eine vorgegebene Differenz ε auseinander (Abbruchkriterium), sind die gesuchten $f_{low}$ und $f_{high}$ gefunden.

Mit den so gefundenen Anteilsfaktoren f lässt sich die molare Masse der Alkane nach Gleichung (11) berechnen. Die äquivalente Kettenlänge wird in einem Schritt 160 (auch mit e) bezeichnet) mit Gleichung (13) gebildet. Sie beträgt:

$$n = \frac{M_{\mathrm{CH}} - 2 * M_{\mathrm{H}}}{M_{\mathrm{C}} + 2 * M_{\mathrm{H}}} \qquad (13)$$

Die in Gleichung (13) enthaltenen molaren Massen von Wasserstoff und Kohlenstoff sind bekannt und können aus Literaturwerten entnommen werden.

In einem letzten Schritt 170 (auch mit f) bezeichnet) kann mit der effektiven Kettenlänge der Emissionsfaktor mit Gleichung (8) in Verbindung mit (6) und (7) berechnet werden. Kennt man den Gasdurchfluss, kann mit dem Emissionsfaktor der CO2-Ausstoß berechnet werden.

Im Folgenden wird ein erfindungsgemäßes Durchflussmessgerät 10 beschrieben, mit dem das erfindungsgemäße Verfahren durchgeführt und aus einem gemessenen Gasdurchfluss und dem Emissionsfaktor e der CO2-Ausstoß berechnet werden kann.

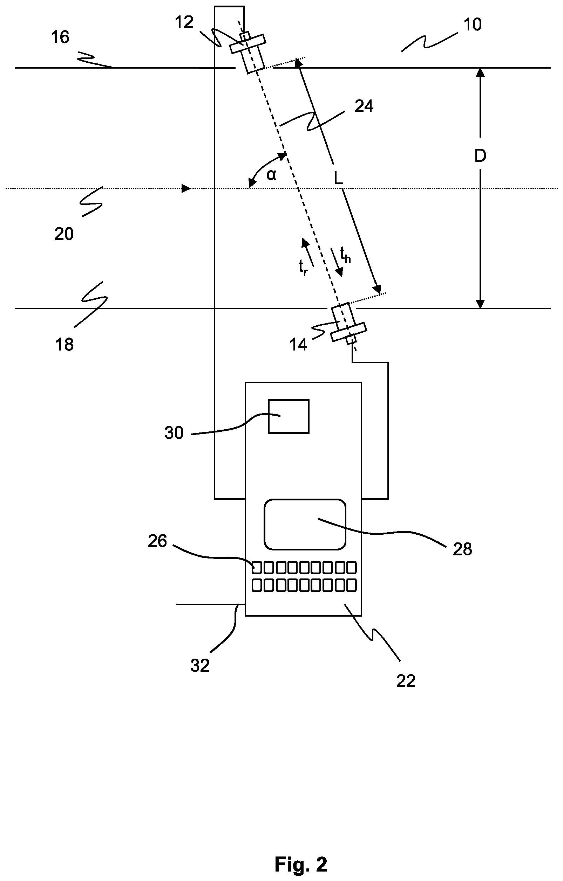

Das erfindungsgemäße Durchflussmessgerät 10 arbeitet nach dem Prinzip der Durchflussmessung mittels Ultraschall. Dieses bekannte Messprinzip ist in Fig. 2 dargestellt. Das Durchflussmessgerät 10 umfasst zwei Ultraschallwandler 12, 14, die in einem Winkel in der Wandung einer Rohrleitung 16 angeordnet sind, in der ein Fluid 18 in Pfeilrichtung 20 strömt. Die Ultraschallwandler 12, 14 arbeiten, von einer Steuer- und Auswertungseinheit 22 angesteuert, wechselweise als Sender und Empfänger. Die auf einem Messpfad 24 durch das Fluid 18 transportierten Ultraschallsignale werden in Strömungsrichtung beschleunigt und gegen die Strömungsrichtung abgebremst. Über nicht dargestellte Schaltungselemente, wie Verstärker und A/D-Wandler, werden der Steuer- und Auswertungseinheit 22 die jeweiligen Empfangssignale zugeführt und digital ausgewertet. Dazu wird die resultierende Laufzeitdifferenz gemäß

$$v = \frac{L}{(2 \cos \alpha)\left(\frac{t_r * t_h}{t_h - t_r}\right)} \qquad (14)$$

zu der gesuchten Strömungsgeschwindigkeit beziehungsweise gemäß

$$\dot{Q} = v \frac{1}{4} D^2 \pi \qquad (15)$$

zu dem Volumenstrom verrechnet, worin die geometrischen Verhältnisse wie in Fig. 2 durch die folgenden Variablen beschrieben sind:

> v: Strömungsgeschwindigkeit des Fluids in der Leitung
> L: Länge des Messpfades zwischen den beiden Ultraschallwandlern
> $\alpha$: Winkel, unter dem die Ultraschallwandler senden und empfangen
> Q: Volumenstrom
> D: Durchmesser der Leitung
> th : Laufzeit des Ultraschalls mit der Strömung
> tr : Laufzeit des Ultraschalls gegen die Strömung

Es versteht sich, dass mit dieser Anordnung auch die Schallgeschwindigkeit des durchfließenden Gases bestimmt werden kann nach

$$SoS = \frac{2L}{(t_h + t_r)} \ .$$

Die Auswerteeinheit 22 umfasst Eingabemittel 26, zum Eingeben der notwendigen Parameter, wie die Anteilsfaktoren $f_{N2}$, $f_{H2O}$, $f_{CO2}$, eine Anzeigeeinheit 28 zum Anzeigen u. a. ermittelter oder gemessener Ergebnisse, wie die Schallgeschwindigkeit SoS und den Durchfluss, sei es Volumendurchfluss $\dot{Q}$ oder Massendurchfluss $\dot{m}$, sowie eine Emissionsfaktorberechnungseinheit 30, in der das erfindungsgemäße Verfahren abläuft. Eine solche Vorrichtung 10 kann nicht nur das erfindungsgemäße Verfahren ausführen, sondern auch die Messung der Schallgeschwindigkeit des Fackelgases vornehmen.

Mit dem erfindungsgemäßen Verfahren und der Vorrichtung lässt sich letztendlich mit Gasdurchfluss und Emissionsfaktor der CO2-Ausstoß nach Gleichung (10) berechnen und kontinuierlich z. B. an einem Ausgang 32 ausgeben.

Ähnlich zu dem oben beschriebenen Verfahren ließe sich bei bekannter äquivalenter Kettenlänge n ein unbekannter Stickstoffanteil $f_{N2}$, was in der Praxis vorkommen kann, bestimmen. Dann würde ein solches Verfahren die folgenden Schritte umfassen:

a) Messen der Schallgeschwindigkeit im Fackelgas,
b) Erfassen von bekannten Anteilen an CO2 und H2O,
c) Berechnen des Kohlenwasserstoffanteils aus der äquivalenten Kettenlänge,
d) Variieren des Stickstoffanteils, solange bis die mit diesem Anteil berechnete Schallgeschwindigkeit innerhalb einer vorgegebenen Differenz zur gemessenen Schallgeschwindigkeit liegt,
e) Berechnen des Emissionsfaktors

## Patentansprüche

1.  Verfahren zur Bestimmung des CO2-Emissionsfaktors bei Fackelgasanlagen mit den Schritten:

    a) Messen der Schallgeschwindigkeit ($SoS_m$) im Fackelgas,
    b) Erfassen von bekannten Anteilen ($f_{N2}$, $f_{H2O}$, $f_{CO2}$) an N2, CO2 und H2O des Fackelgases,
    c1) Berechnen einer maximalen Schallgeschwindigkeit ($SoS(f_{high} = 0)$) des Fackelgases unter der Annahme, dass der Kohlenwasserstoffanteil ($f_{CH}$) des Fackelgas nur aus Alkanen mit Kettenlänge i besteht,
    c2) Berechnen einer minimalen Schallgeschwindigkeit ($SoS(f_{low} = 0)$) unter der Annahme, dass der Kohlenwasserstoffanteil ($f_{CH}$) des Fackelgas nur aus Alkanen mit Kettenlänge i+1 besteht,
    c3) Variieren der Kettenlänge i so lange, bis die gemessene Schallgeschwindigkeit ($SoS_m$) zwischen berechneter minimaler und berechneter maximaler Schallgeschwindigkeit liegt,
    d) Variieren der Anteile ($f_{low}$ und $f_{high}$) der Alkane mit der in den Schritten c1) bis c3) ermittelten Kettenlänge i und der Kettenlänge i+1, so lange, bis die mit diesen Anteilen ($f_{low}$ und $f_{high}$) berechnete Schallgeschwindigkeit ($SoS$) innerhalb einer vorgegebenen Differenz ($\varepsilon$) zur gemessenen Schallgeschwindigkeit ($SoS_m$) liegt,
    e) Berechnen der äquivalenten Kettenlänge (n),
    f) Berechnen des Emissionsfaktors (e) aus den molaren Massen der Anteile des Flaregases und der äquivalenten Kettenlänge nach

$$e * \Omega = \frac{M_{CO2}}{M_{Flare}} * (nf_{CH})$$

wobei $\Omega$ ein Oxydationsfaktor, $M_{CO2}$ die molare Masse von $CO_2$ und $M_{Flare}$ die molare Masse des Flaregases ist.

2.  Verfahren nach Anspruch 1, **dadurch gekennzeichnet, dass** in den Schritten C1) bis C3) die Kettenlänge in iterativen Schritten startend bei i=1 ermittelt wird.

3.  Verfahren nach einem der vorhergehenden Ansprüche, **dadurch gekennzeichnet, dass** in Schritt d) die Anteile in iterativen Schritten ermittelt werden.

4.  Verfahren nach einem der vorhergehenden Ansprüche, **dadurch gekennzeichnet, dass** die Berechnung der Schallgeschwindigkeit nach dem GERG-2004 XT08 Algorithmus erfolgt.

5.  Verfahren nach einem der vorhergehenden Ansprüche, **dadurch gekennzeichnet, dass** die Messung der Schallgeschwindigkeit mit einem Ultraschalldurchflussmessgerät erfolgt.

6.  Verfahren nach einem der vorhergehenden Ansprüche, **dadurch gekennzeichnet, dass** mit einem Ultraschalldurchflussmessgerät der Gasdurchfluss bestimmt wird und mit dem Durchfluss und dem Emissionsfaktor der CO2-Ausstoß berechnet wird.

7.  Verfahren nach Anspruch 6, **dadurch gekennzeichnet, dass** zur Berechnung der Dichte des Fackelgases die gemessene Schallgeschwindigkeit benutzt wird.

**8.** Ultraschalldurchflussmessgerät (10) zur Durchführung eines Verfahrens nach einem der vorhergehenden Ansprüche, mit einer Eingabeeinheit (26) zum Eingeben der Anteile ($f_{N2}$, $f_{H2O}$, $f_{CO2}$) an N2, CO2 und H2O, mit Ultraschallwandlern (12 und 14) zum Aussenden und Empfangen von Ultraschallsignalen, mit einer Auswerteeinheit (22), die ausgebildet ist, um eine Schallgeschwindigkeit ($SoS_m$) zu messen, wobei die Schallgeschwindigkeit aus den Laufzeiten der Ultraschallsignale ermittelbar ist, und die Auswerteeinheit (22) eine Emissionsfaktorberechnungseinheit (30) aufweist, die ausgeführt ist, die Schritte a) bis f) nach Anspruch 1 auszuführen und die Auswerteeinheit (22) weiter ausgebildet ist, um den Gasdurchfluss über die gemessene Strömungsgeschwindigkeit (v) zu bestimmen, wobei die Strömungsgeschwindigkeit (v) aus der Laufzeitdifferenz zweier Ultraschallsignale ermittelbar ist, die einmal eine Komponente mit und einmal eine Komponente gegen die Strömung aufweisen.

**Claims**

**1.** A method for the determination of the $CO_2$ emission factor in flare gas plants comprising the steps of:

a) measuring the speed of sound ($SoS_m$) in the flare gas;
b) detecting known proportions ($f_{N2}$, $f_{H2O}$, $f_{CO2}$) of $N_2$, $CO_2$ and $H_2O$ of the flare gas;
c1) calculating a maximum speed of sound ($SoS(f_{high} = 0)$) of the flare gas under the assumption that the hydrocarbon proportion ($f_{CH}$) of the flare gas only consists of alkanes having a chain length i;
c2) calculating a minimal speed of sound ($SoS(f_{low} = 0)$) under the assumption that the hydrocarbon proportion ($f_{CH}$) of the flare gas only consists of alkanes having a chain length i+1;
c3) varying the chain length i for so long until the measured speed of sound ($SoS_m$) lies between the calculated minimal speed of sound and the calculated maximum speed of sound;
d) varying the proportions ($f_{low}$ and $f_{high}$) of the alkanes having the chain length i and the chain length i+1 determined in steps c1) to c3) for so long until the speed of sound ($SoS$) calculated with these proportions ($f_{low}$ and $f_{high}$) lies within a predefined difference ($\varepsilon$) from the measured speed of sound ($SoS_m$);
e) calculating the equivalent chain length (n); and
f) calculating the emission factor (e) from the molar masses of the flare gas and from the equivalent chain length according to

$$e * \Omega = \frac{M_{CO2}}{M_{Flare}} * (nf_{CH})$$

wherein $\Omega$ is an oxydization factor, $M_{CO2}$ the molar mass of $CO_2$ und $M_{Flare}$ is the molar mass of the flare gas.

**2.** The method in accordance with claim 1, **characterized in that** the chain length is determined in iterative steps c1) to c3) starting at i=1.

**3.** The method in accordance with any preceding claim, **characterized in that** the proportions are determined in iterative steps in step d).

**4.** The method in accordance with any preceding claim, **characterized in that** the step of calculating the speed of sound takes place in accordance with the GERG-2004 XT08 algorithm.

**5.** The method in accordance with any preceding claim, **characterized in that** the step of measureing the speed of sound takes place using an ultrasound flow measurement unit.

**6.** The method in accordance with any preceding claim, **characterized in that** the gas flow is determined by using an ultrasound flow measurement unit and the $CO_2$ emission is calculated by using using the flow and the emission factor.

**7.** The method in accordance with claim 6, **characterized in that** the the measured speed of sound is used for the calculation of the density of the flare gas.

**8.** An ultrasound flow measurement unit (10) for the carrying out of a method in accordance with any preceding claim, having an input unit (24) for inputting the proportions ($f_{N2}$, $f_{H2O}$, $f_{CO2}$) of $N_2$, $CO_2$ and $H_2O$, having ultrasonic transducers (12 and 14) for transmitting and receiving ultrasonic signals, having an evaluation unit (22) which is configured to measure a speed of sound ($SoS_m$) wherein the speed of sound is determined from the time-of-flight of the ultrasound signals and the evaluation unit (22) comprises an emission factor calculating unit (30) which is configured to carry out the steps a) to f) in accordance with claim 1, and the evaluation unit (22) is further configured to determine the gas flow via the measured flow speed (v), wherein the flow speed (v) can be determined from the time of flight difference of two ultrasonic signals which have one component with the flow, on the one hand, and one component against the flow, on the other hand.

## Revendications

1. Procédé pour la détermination du facteur d'émission de CO2 dans des installations de gaz de torchère, comprenant les étapes consistant à :

a) mesurer la vitesse du son ($SoS_m$) dans le gaz de torchère,

b) détecter des parts connues ($f_{N2}$, $f_{H2O}$, $f_{CO2}$) de N2, CO2 et H2O du gaz de torchère,

c1) calculer une vitesse maximum du son ($SoS(f_{high} = 0)$) du gaz de torchère en supposant que la part d'hydrocarbures ($f_{CH}$) du gaz de torchère est constituée uniquement d'alcanes avec des chaînes de longueur i,

c2) calculer une vitesse minimale du son ($SoS(f_{low} = 0)$) en supposant que la part d'hydrocarbures ($f_{CH}$) du gaz de torchère est constituée uniquement d'alcanes avec des chaînes de longueur i +1,

c3) faire varier la longueur de chaîne i aussi longtemps que la vitesse mesurée du son ($SoS_m$) est située entre la vitesse minimale calculée et la vitesse maximale calculée du son,

d) faire varier les parts ($f_{low}$ et $f_{high}$) des alcanes avec des chaînes de longueur i et des chaînes de longueur i +1 déterminées dans les étapes c1) à c3) aussi longtemps que la vitesse du son (SoS) calculée avec ces parts ($f_{low}$ et $f_{high}$) tombe à l'intérieur d'une différence prédéterminée ($\varepsilon$) par rapport à la vitesse mesurée du son ($SoS_m$),

e) calculer la longueur de chaîne équivalente (n),

f) calculer le facteur d'émission (e) à partir des masses molaires des parts du gaz de torchère et la longueur de chaîne équivalente selon l'équation

$$e * \Omega = (M_{CO2}/M_{torchère}) * (nf_{CH}),$$

dans laquelle W est un facteur d'oxydation, $M_{CO2}$ est la masse molaire de CO2 et $M_{torchère}$ est la masse molaire du gaz de torchère.

2. Procédé selon la revendication 1, **caractérisé en ce que** dans les étapes c1) à c3) la longueur de chaîne est déterminée par étapes itératives en commençant par i = 1.

3. Procédé selon l'une des revendications précédentes, **caractérisé en ce que** dans l'étape d) on détermine les parts par étapes itératives.

4. Procédé selon l'une des revendications précédentes, **caractérisé en ce que** le calcul de la vitesse du son est effectué selon l'algorithme GERG-2004 XT08.

5. Procédé selon l'une des revendications précédentes, **caractérisé en ce que** la mesure de la vitesse du son est effectuée avec un appareil de mesure à ultrasons à flux traversant.

6. Procédé selon l'une des revendications précédentes, **caractérisé en ce que** le débit de gaz est déterminé avec un appareil de mesure à ultrasons à flux traversant, et **en ce que** l'on calcule la quantité de CO2 expulsée au moyen du débit et du facteur d'émission.

7. Procédé selon la revendication 6, **caractérisé en ce que** l'on utilise la vitesse mesurée du son pour calculer la densité du gaz de torchère.

8. Appareil de mesure à ultrasons à flux traversant (10) pour mettre en oeuvre un procédé selon l'une des revendications précédentes, comprenant une unité d'entrée (26) pour entrer les parts ($f_{N2}$, $f_{H2O}$, $f_{CO2}$) de N2, CO2 et H2O, avec des transducteurs à ultrasons (12 et 14) pour émettre et recevoir des signaux ultrasonores, comprenant une unité d'évaluation (22) qui est réalisée afin de mesurer une vitesse du son ($SoS_m$), dans lequel la vitesse du son est déterminée à partir des temps de parcours des signaux ultrasonores, et l'unité d'évaluation (22) comprend une unité de calcul de facteur d'émission (30), laquelle est réalisée pour exécuter les étapes a) à f) selon la revendication 1, et l'unité d'évaluation (22) est en outre réalisée pour déterminer le débit de gaz au moyen de la vitesse d'écoulement mesurée (v), ladite vitesse d'écoulement (v) étant susceptible d'être déterminée à partir de la différence de temps de parcours de deux signaux ultrasonores, qui présentent une composante dirigée dans le sens de l'écoulement et une composante dirigée en sens opposé.

äquivalente Kettenlänge

$$M_{CH} = f_{low}(iM_C + (2i+2)M_H) + f_{high}((i+1)M_C + (2(i+1)+2)M_H)$$

$$n = \frac{M_{CH} - 2 * M_H}{M_C + 2 * M_H}$$

**Fig. 1**

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7752884 B


**In der Beschreibung aufgeführte Nicht-Patentliteratur**

- 28th International North Sea Flow Measurement Workshop 2010. **VON FROYSA.** Konferenzband. Energy Institute, POD Publ: Curran Associates, Inc, April 2011, 145
- **VON JEFF GIBSON ; RICHARD PATON ; PAL JAGHO ; KJELL-EIVIND FROYSA ; ANDERS HALLANGER ; ENDRE JACOBSEN ; ANDERS LOVOLL JOHANSEN.** *A Cost-Effective Approach on CO2 Emission Factor Estimation for Flare Ultrasonic Metering Systems*

- The GERG-2004 Wide-Range Equation of State for Natural Gases and Other Mixtures. **VON O. KUNZ ; R. KLIMECK ; W. WAGNER ; M. JAESCHKE.** GERG TECHNICAL MONOGRAPH. VDI Verlag GmbH, 2007, vol. 15